# EUROPÄISCHE PATENTSCHRIFT

(11) **EP 2 488 131 B1**
(45) Veröffentlichungstag und Bekanntmachung des Hinweises auf die Patenterteilung: **25.11.2015**
(21) Anmeldenummer: 10751767.4
(22) Anmeldetag: 27.08.2010
(51) Int. Cl.: A61F 5/00

(54) **VERFAHREN ZUM HERSTELLEN EINER STÜTZ- ODER SCHUTZEINRICHTUNG FÜR EINEN KÖRPERTEIL**
METHOD FOR PRODUCING A SUPPORTING OR PROTECTIVE DEVICE FOR A BODY PART
PROCÉDÉ DE FABRICATION D'UN DISPOSITIF DE SOUTIEN OU DE PROTECTION POUR UNE PARTIE DU CORPS

(30) Priorität: 16.10.2009 AT 16332009
(43) Veröffentlichungstag der Anmeldung: 22.08.2012
(73) Patentinhaber: Stonawski, Rudolf, 1070 Wien (AT)
(72) Erfinder: Stonawski, Rudolf, 1070 Wien (AT)
(74) Vertreter: Sonn & Partner Patentanwälte
(86) Internationale Anmeldenummer: PCT/AT2010/000310
(87) Internationale Veröffentlichungsnummer: WO 2011/044595

(56) Entgegenhaltungen:
- WO-A1-94/20020
- WO-A1-2010/054341
- US-A1- 2004 068 337
- US-A1- 2007 016 323
- US-A1- 2007 207 437

## Beschreibung

Die Erfindung betrifft ein Verfahren zum Herstellen einer Stütz- oder Schutzeinrichtung für einen Körperteil, gemäß Anspruch 1.

### Hintergrund der Erfindung

Als Stütz- oder Schutzeinrichtung für einen Körperteil, etwa zur äußeren, mechanischen Fixierung/Unterstützung, beispielsweise im Fall einer Fraktur, einer Zerrung, Prellung oder nach operativer Behandlung von Sehnen- bzw. Bänderrissen oder Frakturen, ist es bekannt, einen Gipsverband am betroffenen Körperteil anzulegen. Ein derartiger Gipsverband hat jedoch zahlreiche Nachteile, wie etwa die lange Aushärtezeit, das hohe Gewicht, die geringe Festigkeit gegenüber einem Brechen, vor allem in den Randbereichen, und das schwierige Anbringen desselben (Multitraumata). Weiters ist ein derartiger Gipsverband unhygienisch, der Patient kann den betroffenen Körperteil nicht waschen, der Gipsverband führt zu Juckreiz und nach längerer Tragezeit auch zu einer gewissen Geruchsbelästigung, und der Heilungsverlauf ist nicht kontrollierbar, da der betroffene Körperteil unter dem Gipsverband verborgen ist.

Vorgeschlagen wurden auch bereits sogenannte Castverbände als ruhigstellende Verbände, wobei ein dehnbares Trägergewebe aus Glasfasern zugrunde liegt. Derartige Castverbände härten nach ca. 30 Minuten aus und sind nach dieser Zeit bereits voll belastbar. Sie sind im Vergleich zu Gipsverbänden auch leichter, bieten eine verbesserte Durchlässigkeit für Luft und Wasserdampf (Schwitzen), abgesehen von der höheren Festigkeit, was dazu führt, dass praktisch auch bei längeren Tragezeiten keine Reparaturen notwendig sind. Das Material ist überdies unempfindlich gegenüber Wasser, und mit speziellen Überzugs- bzw. Unterlagsmaterialen ist sogar ein Duschen oder Baden möglich. Von Nachteil sind jedoch hier die fast unmögliche Trocknung der Haut, die relativ hohen Kosten und die schlechte Lagerfähigkeit, weiters die vergleichsweise problematische Entsorgung und auch die raue Oberfläche, die zu einer Beschädigung von darüber befindlicher Kleidung führen kann. Darüberhinaus enthalten derartige Castverbände möglicherweise allergieauslösende Stoffe.

Als Stützeinrichtung für verletzte bzw. in einer Rehabilitationsphase befindliche Körperteile, insbesondere im Fall von Brüchen oder Zerrungen, werden vielfach auch Orthesen eingesetzt, die im Hinblick auf eine gute Passung in zahlreichen Größen vorliegen bzw. gefertigt werden müssen und auch deshalb, sowie durch den aufwendigen Formenbau, verhältnismäßig kostenaufwendig sind. Hinzu kommt, dass je nach verletztem Körperteil und je nach Größe und Masse der verletzten Person im Hinblick auf das zu tragende Gewicht verschiedene Materialstärken notwendig sind. Diese Faktoren führen demgemäß überdies zu Lagerproblemen in Spitälern.

Aus der EP 162 692 A2 ist ein Stützverband, insbesondere für Finger, bekannt, wobei von einem ringförmigen Basisteil mehrere über den Umfang verteilte Stäbe ausgehen, die sich zur Fingerspitze hin erstrecken, und über denen ringförmige Fixiervorrichtungen angebracht werden. Diese Ring-Fixiervorrichtungen können durch Bänder gebildet sein, die um die Stäbe herum gelegt und zu einem Ring geschlossen werden; dabei ist es auch bekannt, an den Bändern Distanzelemente anzubringen, die zwischen die Stäbe ragen und so die Stäbe in einem vorgegebenen gegenseitigen Abstand halten. Diese Stützeinrichtung ist allerdings nur für einfache Ausbildungen geeignet, nicht jedoch anwendbar, wenn komplexere Körperteil-Formen gegeben sind, wie etwa im Fall eines Unterschenkels, mit den aufgrund der Wade gegebenen starken Querschnittsänderungen entlang des Unterschenkels.

In der GB 2 375 486 A ist weiters eine Stützeinrichtung beschrieben, bei der metallische Längsstützen mit einem Stoffüberzug vorgesehen sind, die mit Klettverschluss-Verbindungsgurten in gewünschten Abständen fixiert werden können. Auch hier ergibt sich, wie bei der vorerwähnten Finger-Stützeinrichtung, der Nachteil, dass eine Anpassung an komplexere Körperteilformungen wenn überhaupt so nur schwer möglich ist.

Eine Fixierung von Stützen in wählbaren Abständen voneinander mit Hilfe von Haltebändern ist weiters in der WO 89/00036 A geoffenbart. Die Haltebänder haben Schlaufen, um die Stützen in gewünschten Positionen hindurch stecken zu können. Dabei sind die Haltebänder aus einem textilen Material hergestellt. Im Übrigen geht es bei dieser bekannten Stützeinrichtung vor allem um die Ausbildung der Stützen als Klapp-Elemente ähnlich Zeltstangen, um so für die Lagerung weniger sperrige Einheiten vorliegen zu haben.

Aus den obenstehenden Ausführungen ergibt sich bereits, dass im vorliegenden Zusammenhang unter dem Begriff "Fixiervorrichtung" Mittel zu verstehen sind, die den oder die Stütz- oder Schutzteile tragen bzw. am Körperteil festhalten, und/oder die den oder die Stütz- oder Schutzteile in der Position, insbesondere in Relation zu einander, fixieren, und dies gilt auch für die vorliegende Erfindung.

Aus der WO 2004/028417 A ist ein flächiges Stützelement aus einem Gewebe mit darauf aufgeklebten Kunststoffrippen bekannt, wobei die Rippen Ösen mit Löchern für Verbindungs- oder Fixiergurte aufweisen. Die Kunststoffrippen dienen zur Versteifung des Gewebes, welches um den jeweiligen Körperteil herum angebracht wird. Aufgrund der zueinander parallelen Rippen ist auch hier eine Versorgung eines komplexer geformten Körperteils nur schwer möglich. Abgesehen davon wird durch das Gewebe wiederum ein Luftzutritt zum Körperteil beeinträchtigt, und das Gewebe steht auch einem Waschen bzw. Baden entgegen.

Nachteilig ist bei all den vorbekannten Stützeinrichtungen vor allem auch, dass eine individuelle Anpassung an die verschiedensten Körperteile meistens nicht möglich ist.

In der US 2007/0016323 A1 wird das Einscannen von Körperteilen sowie eine Datenaufbereitung für eine automatische Schienenherstellung für Schienen zur Demobilisierung verletzter Körperteile geoffenbart. Die automatische Herstellung erfolgt mittels Stereolithografie; die Schiene wird also schichtweise aufgebaut.

In der US 2004/068337 A1 ist ein Verfahren zur automatisierten Erstellung von Prothesen- bzw. Orthesenmodellen beschrieben, wobei ein Gliedstumpf des Patienten gescannt wird, um Daten für die Fertigung der jeweiligen Prothese bzw. Orthese zu gewinnen.

Die WO 94/20020 A1 beschreibt ein Verfahren zur Vermessung von Fußeigenschaften in Hinblick auf die Herstellung einer Orthese, wobei die erforderlichen Parameter durch Analyse der von mehreren Videokameras in Echtzeit aufgenommenen dreidimensionalen Videobilder ermittelt werden.

### Zusammenfassung der Erfindung

Es ist nun Aufgabe der Erfindung, ein Verfahren wie eingangs angegeben vorzuschlagen, mit dem eine Stütz- oder Schutzeinrichtung in effizienter Anpassung an den jeweiligen Körperteil einfach und kostengünstig herstellbar ist; weiters soll die Stütz- oder Schutzeinrichtung dabei derart ausgebildet werden können, dass sie eine Luftzirkulation zulässt, hygienisch und nicht wasserlöslich ist und einfach handhabbar bzw. anlegbar ist, und dass gegebenenfalls ein Heilungsverlauf, wie z.B. der Grad der Abschwellung, der Zustand der Haut, des Verletzungsgebietes, einer Operationsnarbe etc., überprüfbar ist; überdies soll ein Einsatz für möglichst viele verschiedene Körperteile, zu deren Stützung oder Schutz, ermöglicht werden, wobei weiters auch die verschiedenen Körpergrößen und Gewichte nicht von vornherein zu berücksichtigen sein sollen.

Zur Lösung der gestellten Aufgabe sieht die Erfindung ein Verfahren wie in Anspruch 1 definiert vor. Vorteilhafte Ausführungsformen und Weiterbildungen sind in den abhängigen Ansprüchen angegeben.

Beim vorliegenden Verfahren wird der jeweilige Körperteil, also beispielsweise ein Unterarm, ein Oberarm, ein Fuß, ein Unterschenkel, der Nackenbereich, die Hüfte, aber auch der Kopf, etc., von mehreren Seiten, insbesondere in einer zu fixierenden oder zu schützenden Stellung, fotografiert (bzw. werden allgemein vom Körperteil Bilddaten aufgenommen), und mit Hilfe einer an sich bekannten Bildverarbeitungstechnik wird hieraus rechnerisch ein 3D-Modell berechnet, das die Kontur des Körperteils beinhaltet, und das als "Negativkontur" für die Stütz- oder Schutzeinrichtung bzw. deren Stütz- oder Schutzteile herangezogen werden kann. Was die Bildverarbeitungstechnik anlangt, so sind derartige Verfahren beispielsweise unter der Bezeichnung "Structure-from-motion" ("Struktur aus Bewegung") bekannt, und in vielen Gebieten im Einsatz (vgl. auch http://en.wikipedia.org/wiki/structure_from_motion). Derartige Bildverarbeitungstechniken werden zu einer Rekonstruktion bzw. Darstellung von Objekten aus digitalen Bildern, z.B. von Gebäuden, eingesetzt, vgl. beispielsweise den Artikel von Arnold Irschara, Christopher Zach und Horst Bischof, "Towards Wiki-based Dense City Modeling", Computer Vision, ICCV, 2007, IEEE 11th International Conference, 14-21 October 2007, p. 1-8, ISSN: 1550-5499 (Rio de Janeiro) (s. auch: http://www.icg.tugraz.at/pub/Members/irschara/pub_irschara/wikibasedcitymodeling.pdf).

Aus der US 2002/0006217 A1 ist eine vergleichbare Bildverarbeitungstechnik zur Erstellung von virtuellen, dreidimensionalen Modellen, insbesondere Gebissmodellen, bekannt. Die allgemeine Problemstellung, nämlich die Auffindung einer Kontur oder eines Umrisses auf der Basis von entsprechenden Bilddaten, liegt bei der Form-Erfassung der Körperteile, an die die Stütz- bzw. Schutzeinrichtung angepasst werden soll, ebenso wie bei den vorerwähnten bekannten Techniken zugrunde. Mit Hilfe des erhaltenen 3D-Modells kann dann über eine nummerische Steuerung bzw. computerisierte nummerische Steuerung (NC- bzw. CNC-Einrichtung) auf ebenfalls an sich bekannte Weise eine Ansteuerung von Maschinen für die automatische Herstellung der Stütz- bzw. Schutzeinrichtung erzielt werden. In ähnlicher Weise ist in der US 6,725,118 B ein Verfahren zur zentralisierten Schienen-Herstellung unter Verwendung von Bildabtastung (3D-Abtastung) geoffenbart, um eine genaue Vermessung der Konturen des jeweiligen Körperteils zu ermöglichen; auf die eigentliche Herstellung der Schienen wird jedoch nicht eingegangen.

Aus der EP 1 044 648 B1 ist andererseits ein Verfahren zur Vermessung eines Körperbereiches mittels Kamera bekannt, wobei allerdings ein Referenzgegenstand vorgesehen wird, um mit dessen Hilfe sowie aus Konturlinien ein jeweiliges individuelles Modell zu ermitteln.

Im Einzelnen werden beim vorliegenden Verfahren Steuerdaten hergeleitet, die entsprechende Maschinen über Treiber ansteuern, um so die Form-Anpassung des oder der jeweiligen Stütz- oder Schutzteile zu bewerkstelligen. Unter "Form-Anpassung" ist dabei sowohl die Vorgabe von Längen, als auch die Vorgabe von speziellen Formen bzw. Krümmungen und auch von Positionen innerhalb der jeweiligen Stütz- bzw. Schutzeinrichtung zu verstehen.

Insbesondere können im Zuge der Form-Anpassung gegebenenfalls auch entsprechende Steuerdaten aus dem 3D-Modell für eine Anpassung der Fixiervorrichtung an den Körperteil hergeleitet werden. Als Fixiervorrichtung kann z.B. ein Band verwendet werden, das in der Form bzw. Länge angepasst wird, und das bevorzugt mit voneinander beabstandeten Distanzelementen versehen wird, zwischen denen der zumindest eine Stütz- oder Schutzteil eingefügt wird. In der Regel werden mehrere solche Bänder als Halte-, Trag-, Fixier- bzw. Verbindungselemente vorgesehen werden, etwa im Fall einer Stütz- oder Schutzeinrichtung für den Unterschenkel, gegebenenfalls samt Fuß, eines Menschen, wobei dann beispielhaft drei Bänder im Fußbereich und drei bis vier Bänder im Unterschenkelbereich fixiert werden können. Die Distanzelemente können in ihrer Größe und ihren gegenseitigen Abständen mit Hilfe der gewonnenen Steuerdaten bestimmt werden, und sie werden z.B. im vorgegebenen Abstand am Band befestigt, beispielsweise aufgeklebt und/oder aufgeklipst. Alternativ dazu können diese Distanzelemente auch in der gewünschten Größe und in den gewünschten Abständen am Band, z.B. durch Sägen bzw. Ausfräsen, hergestellt werden. Als Material für das Band oder die Bänder wird bevorzugt ein geeigneter Kunststoff verwendet. Das Band selbst kann in seinen beiden Endbereichen an sich herkömmliche Verschlusselemente zum Schließen des Bandes in der Ringform aufweisen; insbesondere können hier Hakenverschlüsse und dergl., aber auch Klettverschlüsse verwendet werden.
Zur Bildung der Fixiervorrichtung kann auch ein an den Körperteil angepasstes Flächengebilde, z.B. ein textiles Gewebe, aber auch eine Kunststoffunterlage etc., angefertigt werden. Ein derartiges Flächengebilde kann auch direkt zum Tragen des oder der Stütz- oder Schutzteile herangezogen werden, es ist aber auch möglich, ein solches Flächengebilde in Verbindung mit den vorerwähnten Bändern zu verwenden und das Flächengebilde dann beispielsweise als dem Körperteil zugewandte Lage einzusetzen; letzteres ist vor allem dann interessant, wenn es um eine nur für kurze Zeit und vorübergehend anzulegende Schutzeinrichtung geht, wobei das Flächengebilde dann auch eine dämpfende Schaumstofflage oder dergleichen sein kann.

Das Flächengebilde kann somit als Unterlage für den oder die Stütz- oder Schutzteile, gegebenenfalls samt Fixier-Band, angefertigt werden.

Bei einer vorteilhaften Ausführungsform, bei der im Allgemeinen keine Fixier-Bänder erforderlich sind, wird das Flächengebilde mit einer oder mehreren Taschen versehen, in die der oder die Stütz- oder Schutzteile eingesteckt wird bzw. werden. Die Positionen der Taschen können dabei wiederum automatisch, ähnlich wie im Fall der Verwendung von Bändern die Positionen der Stütz- oder Schutzteile an den Bändern, gegebenenfalls unter Trennung durch die Distanzelemente, bestimmt werden. Die Stütz- oder Schutzteile können dann entsprechend einer vorhandenen Zuordnung, etwa von einer Nummerierung oder sonstigen Kennzeichnung, händisch in die jeweiligen zugehörigen Taschen eingesteckt werden, theoretisch kann dies jedoch ebenfalls automatisiert geschehen.

Die Fixiervorrichtung kann weiters mit gesonderten Fixier-Gurten oder anderen Verschlussteilen, wie Hakenverschlüssen, Gummibändern oder dergleichen, zum Festmachen der Stütz- oder Schutzeinrichtung am jeweiligen Körperteil ausgerüstet werden.

Beim Aufnehmen der Bilddaten können auch Röntgenbilder des betroffenen Körperteils aufgenommen werden, was insbesondere bei Knochenbrüchen und dergl. Verletzungen von Vorteil ist, wobei aus diesen Röntgenaufnahmen gewonnene Daten zusätzlich im Hinblick auf die Ausbildung der Stützeinrichtung, etwa was die Dimensionierungen der einzelnen Komponenten, die Dichte der Anordnung der Stützteile etc. anlangt, genützt werden können.

Als Stütz- oder Schutzteile werden stab- bzw. leistenförmige, plastisch verformbare Stütz- bzw. Schutzelemente eingesetzt, die auf Basis der hergeleiteten Steuerdaten bzw. Treibersignale maschinell, automatisch, verformt werden, um die entsprechende Anpassung an die Außenform (Kontur) des jeweiligen Körperteils rechnergestützt vorzunehmen. Die Stütz- bzw. Schutzelemente können auf Basis der Steuerdaten von einem (z.B. extrudierten) Stangenmaterial maschinell in der jeweils erforderlichen Länge abgeschnitten werden, bevor sie verformt werden oder nachdem sie verformt wurden. Weiters können die Stütz- oder Schutzelemente, die z.B. aus einem entsprechenden Kunststoffmaterial, aus einem Laminat oder aus Metall bestehen, einfach durch Anwendung von Hitze und Druck, gegebenenfalls aber auch mit Hilfe von Säge- bzw. Fräseinrichtungen auf Basis der Steuerdaten maschinell umgeformt werden. All diese Schneid- und Formgebungsvorgänge können vollständig automatisch, ohne manuelle Tätigkeiten, ablaufen, wobei es allerdings zweckmäßig sein kann, direkt - im Zuge der Herleitung der Steuerdaten - etwaige individuelle Eingaben (z.B. durch einen Arzt) zusätzlich zu ermöglichen, um beispielsweise gezielt Vorgaben, wie Aussparungen für (geplante) Wunden bzw. Wundnarben, und die Anzahl der Stütz- oder Schutzelemente, nachstehend auch kurz Passelemente genannt, um einen Körperteil herum, machen zu können.

Im Zuge der erfindungsgemäßen automatischen Maschinensteuerung aufgrund der durch die Bildverarbeitung gewonnenen Daten bzw. Treibersignale können Parameter, wie Temperatur und Dauer eines Erwärmungsvorgangs, anzuwendende Drücke beim jeweiligen Verformungsvorgang, abhängig vom verwendeten Kunststoffmaterial, aber auch Kühltemperaturen und Kühlzeiten, sowie Positionen für die Schneid-, Säge- bzw. Fräsvorgänge rechnerisch bestimmt werden. Von Vorteil ist es hier überdies, wenn die Passelemente zwecks Festlegung ihrer Position relativ zur Fixiervorrichtung auf Basis der Steuerdaten automatisch, z.B. durch Prägen, Bedrucken oder Etikettieren, markiert werden.

Nach den einzelnen Formvorgängen können die Stütz- oder Schutzelemente z.B. am jeweiligen Band zwischen den jeweiligen Distanzelementen befestigt werden; hierzu können beispielsweise Schnappverbindungselemente (die zuvor z.B. maschinell und positionsgerecht angebracht wurden) an den Stütz- oder Schutzelementen sowie an den Bändern, zwischen den Distanzelementen, vorhanden sein, um so die Stütz- bzw. Schutzelemente auf der dem Körperteil zugewandten Seite an den Bändern aufzuschnappen oder aufzuklipsen; eine andere (oder ergänzende) Möglichkeit der Befestigung wäre selbstverständlich das Ankleben, gegebenenfalls aber auch Ultraschallschweißen usw.. Im Fall von flächigen Unterlagen können die Passelemente wie erwähnt auch in Taschen oder Laschen eingeschoben werden.

Zum Verformen der - steifen - Passelemente können beispielsweise Formrollen verwendet werden, die unter einem einstellbaren Druck - der durch die Steuerdaten vorgegeben wird - die Passteile bzw. Passelemente sowohl hinsichtlich der Querschnittsform als auch hinsichtlich Krümmungen etc. formen.

Die Passelemente können auf diese Weise einzeln genau entsprechend der Kontur des betroffenen Körperteils geformt werden; die Passelemente bestehen zu diesem Zweck bevorzugt aus einem thermoplastischen Kunststoff.

Die Oberfläche der Passteile, gegebenenfalls auch der Fixier-Bänder und der Distanzelemente, kann vorzugsweise mit einer Lotuseffekt-Nanostruktur ausgebildet werden, um so das Anhaften von Schmutz zu erschweren.

Zur Bilddaten-Aufnahme kann eine digitale Kamera, aber auch eine Phasenkamera und/oder ein Laserscanner, wie an sich bekannt, verwendet werden, und das 3D-Modell kann auch unter Anwendung eines ebenfalls an sich bekannten Konturlinienverfahrens (engl.: "visual hull") erstellt werden , vgl. auch: Aldo Laurentini: The Visual Hull Concept for Silhouette-Based Image Understanding. IEEE Trans. Pattern Anal. Mach. Intell. 16(2): 150-162 (1994).

Von Vorteil ist bei der vorliegenden Vorgangsweise in der besonders bevorzugten Ausführungsform, dass nur zwei verschiedene Grundkomponenten (Kunststoffstrang für die Stütz- oder Schutzteile und Band für die Fixiereinrichtung) erforderlich sind, und dass weiters die Vorgangsweise mit der Erstellung eines 3D-Modells und der automatischen Ansteuerung der Schneid-, Form-, Fräsvorgänge etc. für alle beliebigen Körperteile angewendet werden kann. Ein Röntgenbefund kann wie erwähnt als zusätzliche Informationsquelle eingebunden werden. Was die maschinelle Verformung betrifft, so kann die Einrichtung hierfür verhältnismäßig einfach, z.B. mit bloß drei Formrollen, einer vorhergehenden Heizeinrichtung und eine Druckmechanik, ausgebildet sein. Die Formgebung erfolgt fern vom Körperteil, und es ist somit keine Aushärtungszeit von Komponenten am Körperteil erforderlich. Die hergestellte Stütz- oder Schutzeinrichtung ist leicht, stabil, hygienisch, waschbar, sterilisierbar, und es sind auch Verbände integrierbar. Weiters ist eine offene Ausbildung, mit entsprechenden Lüftungsmöglichkeiten, gegeben. Im Fall einer Stütz- oder Schutzeinrichtung für den Fuß und Unterschenkel kann auch ein "Gehteil" mit einer Abrollfunktion vorgesehen werden, wodurch das Gehen mit der Stütz- oder Schutzeinrichtung komfortabler und schonender wird.

### Kurzbeschreibung der Zeichnungen

Die Erfindung wird nachfolgend anhand von bevorzugten Ausführungsbeispielen, auf die sie jedoch nicht beschränkt sein soll, und unter Bezugnahme auf die Zeichnung auch weiter erläutert. In der Zeichnung zeigen dabei im Einzelnen:
- Fig. 1: schaubildlich eine erfindungsgemäß hergestellte Stützeinrichtung für einen Unterschenkel samt Fuß;
- Fig. 2: in einem Schema die Vorgangsweise bei der Herstellung einer derartigen Stützeinrichtung mit der erfindungsgemäßen individuellen Anpassung;
- Fig. 3: schematisch und schaubildlich eine Anlage zum Umformen von Stützelementen, die von einem Stangenmaterial in den erforderlichen Längen und zwischen Formwalzen in einer vorgegebenen Weise verformt, geschnitten, gesägt bzw. gefräst, mit Verbindungsteilen (z.B. Klips oder Klebstoff) versehen und markiert werden;
- Fig. 4: schaubildlich einen Teil eines als Fixiervorrichtung vorgesehenen Bandes mit pyramidenstumpfförmigen Distanzelementen;
- Fig. 5: schaubildlich das Einklipsen eines stabförmigen Stützelements, wie gemäß Fig. 3 erhalten, zwischen zwei Distanzelementen auf einem Band gemäß Fig. 4;
- Fig. 6: in einer Abwandlung der Ausführung gemäß Fig. 5 das Anbringen eines mit im Querschnitt dreieckigen, seitlichen Kerben versehenen stabförmigen Stützelements zwischen zwei gegenüber der Ausrichtung gemäß Fig. 5 verdrehten pyramidenstumpfförmigen Distanzelementen auf einem Band;
- Fig. 7: in schaubildlicher Darstellung ähnlich Fig. 5 und 6 eine weitere Ausführungsform eines nur teilweise dargestellten stabförmigen Stützelements, hier mit äußeren Rippen oder Noppen, die in entsprechende Ausnehmungen von Distanzelementen auf einem Band passen;
- Fig. 8: eine nach dem erfindungsgemäßen Verfahren hergestellten Schutzeinrichtung in Form eines Schienbeinschützers, z.B. für Fußballer, in einer Ansicht ähnlich Fig. 1;
- Fig. 9: in einer den Fig. 1 und 8 vergleichbaren Ansicht einen Bein-Protektor, z.B. für Motorradfahrer, als Schutzeinrichtung;
- Fig. 10: eine weitere Schutzeinrichtung in Form eines Ellbogen-Protektors, z.B. für Zweiradfahrer oder Rollschuhläufer, in schaubildlicher Ansicht;
- Fig. 11: eine Schutzeinrichtung in Form eines Schutzhelms, z.B. für Motorradfahrer, Eishockey-Spieler oder Football-Spieler;
- Fig. 11A: eine Detailansicht zu Fig. 11, zur Veranschaulichung der Anbringung von einzelnen angepassten Schutzteilen des Helms von Fig. 11 in an einer Innenlage angebrachten Taschen;
- Fig. 12: in einer Ansicht ähnlich Fig. 11 eine Schutzmaske, z.B. für einen Eishockey-Tormann;
- Fig. 13: eine Ansicht von Körper-Schutzteilen, z.B. für einen Football-Spieler;
- Fig. 13A: eine Detailansicht zu Fig. 13, zur Veranschaulichung des Einschiebens von angepassten stangenförmigen Schutzteilen in auf einem Flächengebilde, z.B. textilem Gewebe, angenähte Taschen;
- Fig. 14: schaubildlich Schutzteile an einer Schutzhose, z.B. als Schutzkleidung für Arbeiter oder Sportler;
- Fig. 14A: eine Detailansicht zu Fig. 14, zur Veranschaulichung des Einschiebens von angepassten stangenförmigen Schutzteilen in Gewebetaschen;
- Fig. 15: eine Stützeinrichtung in Form eines Korsetts oder Miederteils, mit herkömmlichem rückseitigem Verschluss; und
- Fig 15A: in einer Detailansicht zu Fig. 15 das Unterbringen von angepassten stangenförmigen Stützelementen in an das Gewebe des Korsetts oder Miederteils genähten Taschen.

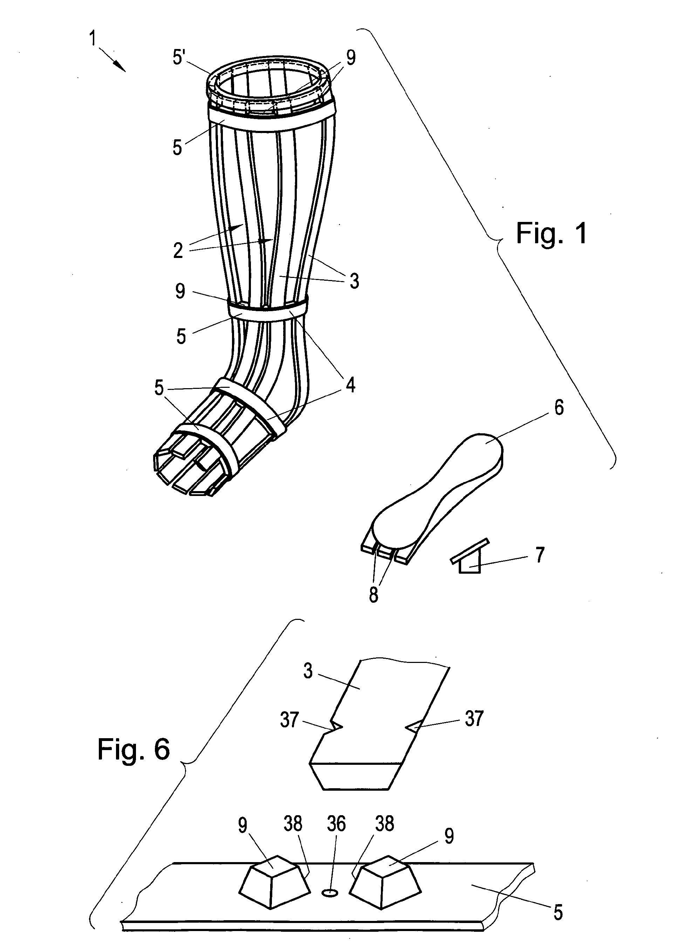

Detaillierte Beschreibung bevorzugter Ausführungsformen In Fig. 1 ist beispielhaft und schematisch eine Stützeinrichtung 1 zur Anwendung an einem Unterschenkel und Fuß einer Person veranschaulicht, wobei die Stützeinrichtung 1 als eigentliche Stützteile 2 mehrere über den Umfang des Unterschenkels und des Fußes verteilte längliche, stab- bzw. leistenförmige entsprechend gebogene Stützelemente 3 enthält. Diese Stützelemente 3 werden mit Hilfe einer Fixiervorrichtung 4 relativ zueinander und am Unterschenkel bzw. Fuß (nachstehend auch kurz "Körperteil" genannt) fixiert, wobei diese Fixiervorrichtung 4 im gezeigten Beispiel mehrere Bänder 5 aufweist, die um den Körperteil herum ringförmig geschlossen werden, und an denen außen die Stützelemente 3 befestigt sind, wie nachstehend noch näher erläutert wird.

Wenngleich in Fig. 1 langgestreckte Stützelemente 3 als Hauptkomponenten der Stützeinrichtung 1 gezeigt sind, so sollte doch verständlich sein, dass auch streifenförmige oder schalenförmige Stützteile 2, die jeweils einen Teil des Körperteils unter enger Anlage bedecken, denkbar sind. Auch ist es möglich, in diesen schalenförmigen Stützteilen an Stellen, wo eine stützende Anlage am Körperteil nicht notwendig ist, Durchbrechungen zwecks Ermöglichung einer Luftzirkulation vorzusehen.

In Fig. 1 ist in Ergänzung zu den langgestreckten Stützelementen 3 als zusätzlicher Stützteil eine Fußplatte 6 in herausgezogener Darstellung veranschaulicht, die im gezeigten Beispiel den Fuß stützen und schützen soll, und die auch ein Gehen ermöglichen soll. Dieser Fußplatte kann ein an sich üblicher stopfenförmiger Absatzteil 7 (oder anderer üblicher der Abrollbewegung zuträglicher Teil) zugeordnet sein, wie dies an sich von beispielsweise Gipsverbänden durchaus bekannt ist.

Wie weiters aus Fig. 1 zu ersehen ist, hat die Fußplatte 6 an ihrer Vorderseite Einschnitte oder Kerben 8, in die die unteren Enden der Stützelemente 3 der Stützeinrichtung 1 passen, was aber in Fig. 1 nicht näher dargestellt ist.

Die Stützteile 2 bzw. Stützelemente 3 werden beim vorliegenden Verfahren individuell an die Form (Konturen) des Körperteils, also beispielsweise des Unterschenkels und Fußes, angepasst. Dies erfolgt, ausgehend von einer Erfassung der Form oder Konturen des Körperteils, unter Ableitung von entsprechenden Maschinen-Steuerdaten für die Formgebung, Längenbestimmung und Positionsfestlegung, weitestgehend automatisch.

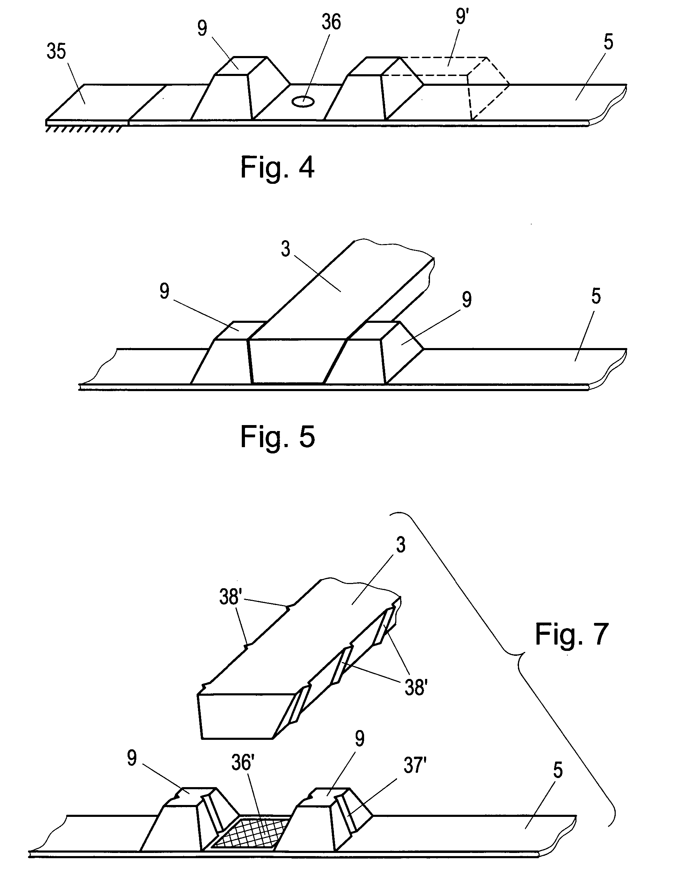

Ähnliches gilt auch für die Bänder 5, die bevorzugt zwischen den Stützelementen 3 Distanzelemente oder Anschlagelemente 9 tragen, wie dies nur ganz schematisch mit einzelnen derartigen Distanzelementen 9 in Fig. 1 bei einzelnen Bändern 5 beispielhaft veranschaulicht ist. Hinsichtlich dieser Distanzelemente wird nachfolgend, insbesondere anhand der Fig. 4 bis 7, noch eine nähere Erläuterung erfolgen.

In Fig. 1 ist auch ein - im Querschnitt beispielsweise U-förmiges - Abschlussband 5' gezeigt, das bevorzugt weich, flexibel ist und auf die oberen Enden der Stützteile 2 aufgesetzt bzw. aufgeschoben und z.B. angeklebt oder angeklipst wird; ggf. kann auch auf die unteren Enden ein solches Abschlussband aufgebracht werden. Das Abschlussband 5' kann z.B. aus einem Band 5 mit Distanzelementen 9 sowie mit einem hautfreundlichen, schmiegsamen Saum gebildet sein, der nach oben absteht und um die Enden der Stützteile 2 herum - nach innen Richtung Körperteil - geschlagen wird.

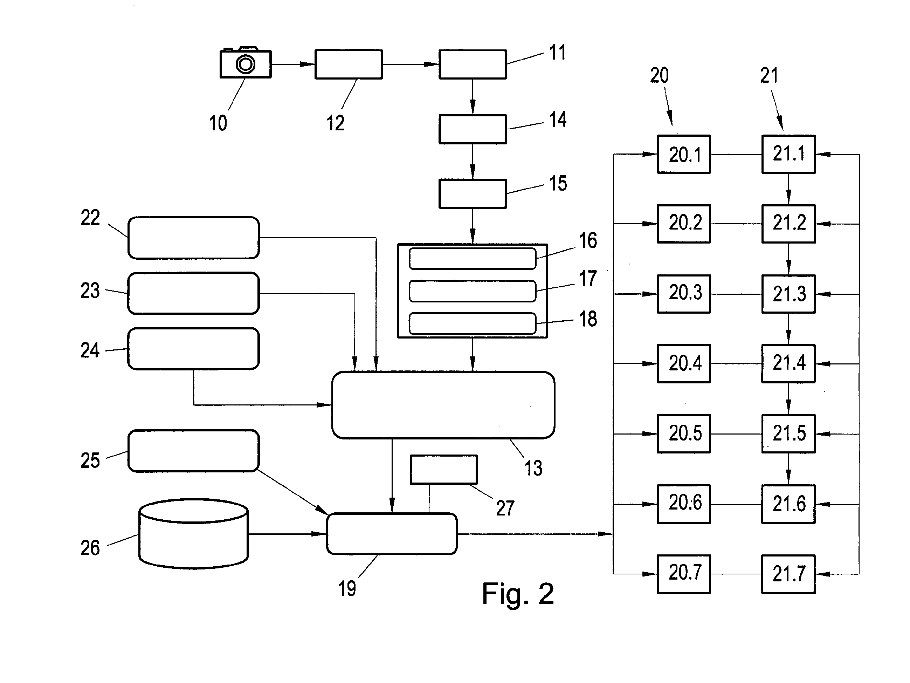

In Fig. 2 ist in einem Schema ein beispielhafter Ablauf für die Vorgangsweise bei der Herstellung einer Stützeinrichtung 1 veranschaulicht, wobei ausgehend von einer Bilderfassung gemäß Schritt 11 mit Hilfe einer Kamera 10, nach deren Kalibrierung 12, ein 3D-Modell 13 in einer an sich bekannten Weise mit Hilfe von Rechnermitteln ermittelt wird. Dabei werden die aufgenommenen Bilddaten in einem Schritt 14 automatisch ausgerichtet, wonach eine Mehrfachbild-Segmentierung in einem Schritt 15 folgt.

Über weitere Bearbeitungsschritte, wie eine 3D-Rekonstruktion 16, eine Dichte-Anpassung 17 und eine volumetrische Integration 18 gelangt man dann zum gewünschten 3D-Modell 13.

Auf Basis dieses 3D-Modells 13 wird sodann über eine nummerische Steuerung bzw. computerisierte nummerische Steuerung (NC bzw. CNC-Steuerung), im Prinzip einen Rechner 19, ein Satz von Steuersignalen für verschiedene Treiberstufen erzeugt, welche in Fig. 2 in ihrer Gesamtheit mit 20 bezeichnet sind. Diese Treiberstufen 20 steuern dann über entsprechende Treibersignale verschiedenste Formgebungsmittel 21 an, wobei hierunter nicht nur die eigentlichen Umformungen der Stützteile 2, gegebenenfalls auch der zur Fixierung vorgesehenen Bänder 5 mit den Distanzelementen 9, fallen, sondern auch andere Vorgänge, wie Schneiden auf die erforderlichen Längen, Erwärmen vor dem Verformen, Kühlen nach dem Verformen, Fräsen im Fall der geeigneten Dimensionierung der Distanzstücke auf den Bändern 5, aber auch das Anbringen von Markierungen auf den Stützteilen 2. Beispielhaft sind in Fig. 2 entsprechende maschinelle Mittel konkret mit 21.1 (Schneideinrichtung), 21.2 (Heizeinrichtung), 21.3 (Umformeinrichtung), 21.4 (Kühleinrichtung), 21.5 (Säge-, Laser- und/oder Fräseinrichtung), bzw. 21.6 (Einrichtung zum Anbringen von Markierungen auf den Stützteilen 2) bzw. 21.7 (Einrichtung zum Anbringen von Verbindungselementen z.B. Klipsen) veranschaulicht, wobei je nach Anforderung und Zielsetzung auch andere bzw. noch weitere Einrichtungen für die Anpassung der Stützteile, Distanzstücke, Bänder, Fußplatten usw. möglich sein können.

Die entsprechenden, diesen Einrichtungen 21 zugeordneten Treiberstufen sind in Fig. 2 im Einzelnen mit 20.1, 20.2 bis 20.7 bezeichnet.

Anstatt eine Digitalkamera 10 zu verwenden, um Bilddaten vom jeweiligen Körperteil, z.B. Unterschenkel samt Fuß, zu erhalten und im Anschluss daran das 3D-Modell 13 zu errechnen, können auch andere Techniken angewendet werden, um zum 3D-Modell 13 zu gelangen. So ist in Fig. 2 mit einem Block 22 veranschaulicht, dass mit einem Konturlinienverfahren vom jeweiligen Körperteil ein 3D-Modell 13 erstellt wird. Weiters können gemäß Block 23 mit Hilfe einer Phasenkamera entsprechende Daten zur Erstellung des 3D-Modells 13 erhalten werden; in entsprechender Weise kann auch gemäß Block 24 ein Laserscanner eingesetzt werden, um ausgehend vom jeweiligen Körperteil durch einen Scannvorgang zu Daten und über diese Daten zur Erstellung des 3D-Modells 13 zu kommen.

Den Rechnermitteln 19, wo im Zuge der NC- bzw. CNC-Steuerung Steuersignale für die Treiberstufen 20 hergeleitet werden, kann auch eine Eingabeeinrichtung 25 für individuelle Eingaben durch einen Arzt, Podologen etc. zugeordnet sein; auf diese Weise können beispielsweise für die Berechnung der Stützteile 2 bzw. Stützelemente 3 auch Parameter wie Masse (Gewicht) und Größe eines Patienten, Alter des Patienten, Korrektur von etwaigen Fehlstellungen, die Zugänglichkeit bzw. Position von Wund- oder Operationsbereichen (zur Wundverlaufs-/Heilungskontrolle) usw. eingegeben werden, um derartige Parameter bei der Ermittlung der Steuersignale für die Treiber 20 und damit der Ansteuerung der Formeinrichtungen 21 zu berücksichtigen.

Weiters ist in Fig. 2 noch eine Datenbank 26 gezeigt, in der beispielsweise auch empirische Parameter oder Daten, z.B. betreffend jeweilige Vorgabe-Positionen von Stützteilen 2 an einem bestimmten Körperteil, abgelegt sein können.

Schließlich ist in Fig. 2 schematisch mit einem Block 27 noch veranschaulicht, dass Röntgenfotos oder Daten einer Computertomographie (CT) ebenfalls bei der Ermittlung der Steuerdaten für die einzelnen Formeinrichtungen 21 mit einbezogen werden können.

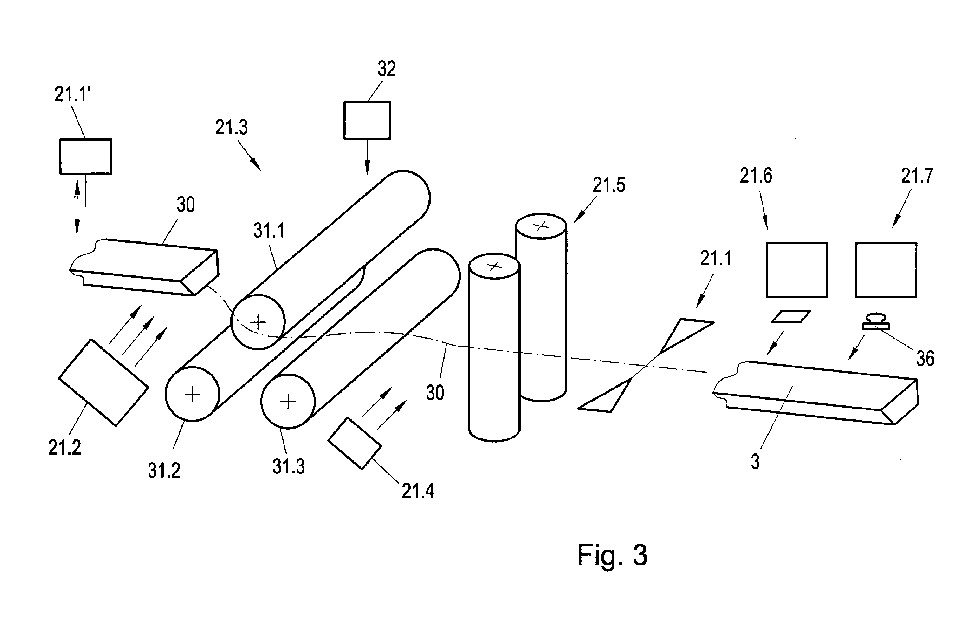

Auf Basis derartiger Röntgen- oder CT-Aufnahmen bzw. Röntgen- oder CT-Daten ebenso wie auf Basis von individuellen Eingaben gemäß Block 25 kann auch ein geeignetes Material für die Stützteile 2 bzw. Stützelemente 3 ausgewählt werden, also beispielsweise ein entsprechend festes bzw. entsprechend dimensioniertes Ausgangsmaterial, wie insbesondere ein Stangenmaterial 30 (s. Fig. 3) im Fall der Herstellung von länglichen Stützelementen 3 gemäß Fig. 1.

Was die Materialien für die Stützteile (oder Schutzteile) 2 im einzelnen betrifft, so sollten diese Materialien ausreichend fest bzw. steif und zäh und so weit thermostabil sein, dass sie bei Heißdampf, bei 134° C, sterilisierbar und überdies chemisch beständig sind. Weiters sollten die Stütz- oder Schutzteile 2 im Hinblick auf die direkte Anlage am jeweiligen Körperteil biokompatibel und schweißbeständig sein. Im Fall von medizinischen Stützeinrichtungen sollte das Material nach Möglichkeit transparent bzw. transluzent gegenüber Röntgenstrahlen und/oder MR-T-transparent oder -beständig sein. Weitere Anforderungen sind Verschleißfestigkeit, Farbechtheit, Dimensionsstabilität und Spannungsrissbeständigkeit.

Demgemäß kommen aus Kunststoff beispielsweise amorphe Thermoplaste, wie PC, oder aber, als Hochtemperaturkunststoffe, PES, PPSU, PEI, PSU und PPE infrage. Als teilkristalline Thermoplaste können beispielsweise PP oder aber, wenn eine Eignung für höhere Temperaturen gewünscht ist, PET, PBT, POM, PVDF und PEEK eingesetzt werden. Zum Warmumformen müssen derartige teilkristalline Thermoplaste etwas über die Kristallitschmelztemperatur aufgeheizt werden; die amorphen Thermoplaste werden für die Umformung über die Glasübergangstemperatur erwärmt. Die erforderlichen Temperaturen sind für die Thermoplaste unterschiedlich und liegen allgemein zwischen 100° C und 350° C. Für sterilisierbare Stützeinrichtungen sind Hochtemperaturkunststoffe zu verwenden. Sofern Metalle eingesetzt werden sollen, kommen Platin und Platinlegierungen, Tantal und Tantallegierungen, Titan und Titanlegierungen, Edelstähle und NiCr-Legierungen, ggfs. auch Al-Legierungen, infrage.

Das Stangenmaterial, von dem die einzelnen Stütz- oder Schutzelemente 2 bzw. 3 abgeschnitten werden, kann auch ein Laminat oder Co-Extrudat sein, wobei auf der der Körperseite zuzuwendenden Seite ein weiches, hautfreundliches Material vorliegen kann, wogegen das im Laminat dahinter (außen) befindliche Material ein steifes Thermoplastmaterial, als thermisch verformbares Stützmaterial, sein wird.

Als Materialien kommen schließlich auch noch Memory-Werkstoffe, also Werkstoffe mit Formgedächtnis, infrage, wie z.B. mit einem Einweg-Memory-Effekt, mit einem Zweiweg-Memory-Effekt, mit einer Formänderung bei Zufuhr von Energie, etwa in Form von elektrischem Strom, oder aber bei Temperaturunterschieden. Von Vorteil können auch sogenannte Aktorwerkstoffe sein, die ein Verhalten ähnlich einem menschlichen Muskel aufweisen können, und durch deren Einsatz z.B. mechanische Gelenke im Bereich der Stütz- oder Schutzeinrichtungen ersetzt werden können.

In Fig. 3 ist nun ganz schematisch veranschaulicht, wie ausgehend von einem geeigneten, d.h. entsprechend dem jeweiligen Anwendungsfall ausgewählten Stangen- bzw. Strangmaterial oder Endlos-Material 30, insbesondere aus thermoplastischem Kunststoff, mit Hilfe der verschiedenen Formeinrichtungen 21 (s. Fig. 2) Stützelemente 3 gemäß Fig. 1 hergestellt werden. Das Stangenmaterial 30 wird dabei nach Erwärmung mit Hilfe der Heizeinrichtung 21.2 einer Umformeinrichtung 21.3 mit Umformwalzen 31.1, 31.2 und 31.3 zugeführt, wobei eine nur schematisch veranschaulichte Druckeinstelleinheit 32 für die obere Umformwalze 31.1 vorgesehen ist, um diese mit dem jeweiligen Druck und auch mit der jeweiligen Verstellung relativ zu den beiden unteren Umformwalzen 31.2, 31.3 zu bewegen und so entsprechende Krümmungen, Biegungen, Einbuchtungen oder dergl. im Stangenmaterial 30 vorzusehen. Selbstverständlich ist es dabei aber auch möglich, eine oder beide der unteren Umformwalzen 31.2, 31.3 relativ zur oberen Umformwalze 31.1 zu bewegen, und insbesondere können auch alle drei (oder mehr) Umformwalzen 31 beweglich in einem Gestell gelagert sowie verstellt und mit entsprechendem Druck beaufschlagbar sein.

Nach Durchlaufen dieser Formstation mit den Umformwalzen 31 wird das Strang- bzw. Stangenmaterial 30 mit Hilfe der Kühleinrichtung 21.4, beispielsweise einem Gebläse, abgekühlt, wonach in einer Säge-, Laser- und/oder Fräseinheit 21.5 ein beispielsweise seitliches Bearbeiten durch Sägen bzw. Fräsen erfolgt; danach werden schließlich in der Schneideinheit 21.1 die einzelnen stangenförmigen Stützelemente 3 abgetrennt. Diese Stützelemente 3 können dann noch mit Hilfe der Markiereinrichtung 21.6 markiert werden, damit ihre erforderliche Position relativ zum Körperteil bzw. zu den Bändern 5 festgehalten wird.

Weiters ist in Fig. 3 schematisch eine Einrichtung 21.7 zum Anbringen von zusammenpassenden Verbindungselementen 36, z.B. Schnapp- oder Klipselementen, auf den Stützelementen 3 und Bändern 5 gezeigt. In Fig. 3 ist schematisch veranschaulicht, wie ausgehend von einem geeigneten, d.h. entsprechend dem jeweiligen Anwendungsfall ausgewählten Material Distanzelemente 9 geformt, geschnitten, gefräst etc. werden können.

In Fig. 4 ist schaubildlich ein Teil eines Bandes 5 mit darauf vorgesehenen Distanzelementen 9 gezeigt, wobei weiters am Bandende ein Klettverschlussabschnitt 35 ersichtlich ist, der mit einem entsprechenden Abschnitt am anderen , in Fig. 4 nicht gezeigten Ende zum Schließen des Bandes 5 um einen Körperteil herum, z.B. einen Unterschenkel oder Fuß gemäß Fig. 1, zusammenarbeitet. Zwischen den in Fig. 4 ersichtlichen zwei Distanzelementen 9 (selbstverständlich sind im Fall des Ausführungsbeispiels der Stützeinrichtung 1 gemäß Fig. 1 weitere Distanzelemente 9 auf jedem Band 5 angeordnet), wird, wie aus Fig. 5 ersichtlich ist, das jeweilige Stützelement 3 formschlüssig eingefügt und befestigt, z.B. angeklebt. Es können jedoch auf dem Band 5 und ebenso auf der Innenseite (Unterseite gemäß Fig. 5) der Stützelemente 3 entsprechende z.B. Druckknopf-artige Klips-, Niet- oder Schnappelemente 36 auf dem Band 5 bzw. den Stützelementen 3, vorgesehen worden sein, vgl. auch die Einrichtung 21.7 in Fig. 2 und 3, um die Stützelemente 3 einfach, gegebenenfalls auch automatisch, in der richtigen Position durch Aufklipsen oder Aufschnappen auf dem Band 5 zu fixieren. In den Darstellungen gemäß Fig. 4 und 5 ist dabei die obere Seite des Bandes 5, die den Stützelementen 3 zugewandt ist, und die die Distanzelemente 9 trägt, die Außenseite des Bandes, die vom jeweiligen Körperteil abgewandt ist; es kann die obere Seite des Bandes 5 aber auch die Innenseite sein, vgl. auch die Darstellung in Fig. 1.

Die Distanzelemente 9 können auf einem jeweiligen Band 5 in gleichen, aber auch in verschiedenen Abständen vorgesehen sein, und sie können auch in ihrer Erstreckung in Längsrichtung des Bandes 5 in Entsprechung zu den erforderlichen Abständen der Stützelemente 3 am jeweiligen Band 5 größer oder kleiner dimensioniert werden, was in Fig. 4 mit strichlierten Linien 9' angedeutet ist. Grund hierfür ist, dass bei einem sich in seiner Querschnittsform ändernden Körperteil, wie etwa einem Unterschenkel, s. Fig. 1, die Stützelemente 3 je nach Position am Körperteil, also Unterschenkel im gegebenen Beispiel, weiter auseinander (im oberen Wadenbereich in Fig. 1) oder aber näher aneinander (oberhalb des Knöchelbereichs in der Darstellung gemäß Fig. 1) zu liegen kommen. Die entsprechende Formgebung der Stützelemente 3 ebenso wie der Distanzelemente 9 auf den Bändern 5 kann automatisch mit Hilfe der Einrichtungen 21 erfolgen. Beispielsweise werden die Distanzelemente 9 mit der entsprechenden Dimension berechnet und ausgewählt sowie anschließend am Band 5 befestigt, z.B. aufgeklipst oder angeklebt, wobei eine entsprechende Maschine die Distanzelemente 9 mit Hilfe der Steuersignale automatisch positioniert und fixiert. Alternativ dazu ist jedoch möglich, die Distanzelemente 9 durch Ausfräsen der Aufnahme-Bereiche zwischen ihnen aus einem anfänglich entsprechend dicken Band 5 vorzusehen, oder aber ursprünglich übergroß (überlang) bemessene Distanzelemente 9, die fix auf dem Band 5 angebracht bzw. geformt sind, auf die richtige Größe zurechtzufräsen.

Gemäß Fig. 6 können zur Verstärkung des Formschlusses zwischen Stützelementen 3 und Distanznoppen 9 die Stützelemente 3 in den Seitenwänden im Querschnitt beispielsweise dreieckige Einschnitte oder Kerben 37 aufweisen, die z.B. beim Fräsvorgang (Fräseinrichtung 21.5) erhalten werden können, und die mit Seitenkanten 38 von entsprechend gegenüber Fig. 4 und 5 um beispielsweise 45° verdreht angeordneten Distanznoppen 9 zusammenwirken.

Alternativ dazu kann gemäß Fig. 7 in den Distanznoppen 9 jeweils eine Kerbe 37' vorgesehen sein, die mit vorstehenden, entsprechend geformten Rippen 38' oder Noppen an den Seitenwänden der Stützelemente 3 zur Erzielung des verstärkten Formschlusses zusammenwirken. Weiters ist als alternative Verbindungsmöglichkeit in Fig. 7 eine auf der dem Stützelement 3 zugewandten Innenseite aufgebrachte Klebeschicht 36' zur Verbindung mit dem Stützelement 3 durch Ankleben veranschaulicht, wobei diese durch die Einrichtung 21.7 (Fig. 3) angebracht werden kann.

Die vorstehend erläuterten und in der Zeichnung gezeigten konkreten Ausführungsformen sind selbstverständlich nur beispielhaft zu verstehen, und es sind weitere Abwandlungen und Modifikationen im Rahmen der Erfindung möglich. So ist es beispielsweise denkbar, im Fall einer ausreichend festen Verbindung zwischen Stützelementen 3 und Bändern 5 durch Aufklipsen und/oder Aufkleben an den gewünschten Positionen die Distanzelemente 9 einfach wegzulassen. Die Stützelemente 3 können auch eine andere Form, insbesondere abweichend vom gezeigten Trapezförmigen Querschnitt, aufweisen. Weiters kann in Abwandlung der Vorgangsweise gemäß Fig. 3 das Stützelement-Stangenmaterial 30 bereits bei der Zuführung zur Formeinrichtung 21.3 auf die entsprechende Länge geschnitten werden, wie dies schematisch in Fig. 3 mit der Schneideinheit 21.1' veranschaulicht ist. Die Markierung der Stützelemente 3 kann ferner durch Aufkleben von Markierungen ebenso wie durch Prägevorgänge oder Druckvorgänge erfolgen. Im Fall von Stützteilen 2 mit einer flachen, streifenförmigen Form, die zu einer Schalenform umzuformen sind, können entsprechend andere, an sich bekannte Umformeinrichtungen anstatt der gezeigten Umformwalzen 31 vorgesehen werden.

Als Material für die Stützteile 2 werden wie erwähnt bevorzugt thermoplastische Kunststoffe, Metall, gegebenenfalls aber auch andere Werkstoffe, mit der entsprechenden Festigkeit, angepasst an den jeweiligen Einsatzzweck verwendet; beispielsweise können Stützteile 2 für einen verletzten Finger aus einem weniger festen Material bestehen als im Fall von den Stützteilen 2 bei einem Schienbeinbruch, wobei sich dann die Stützteile über Unter- und Oberschenkel erstrecken würden.

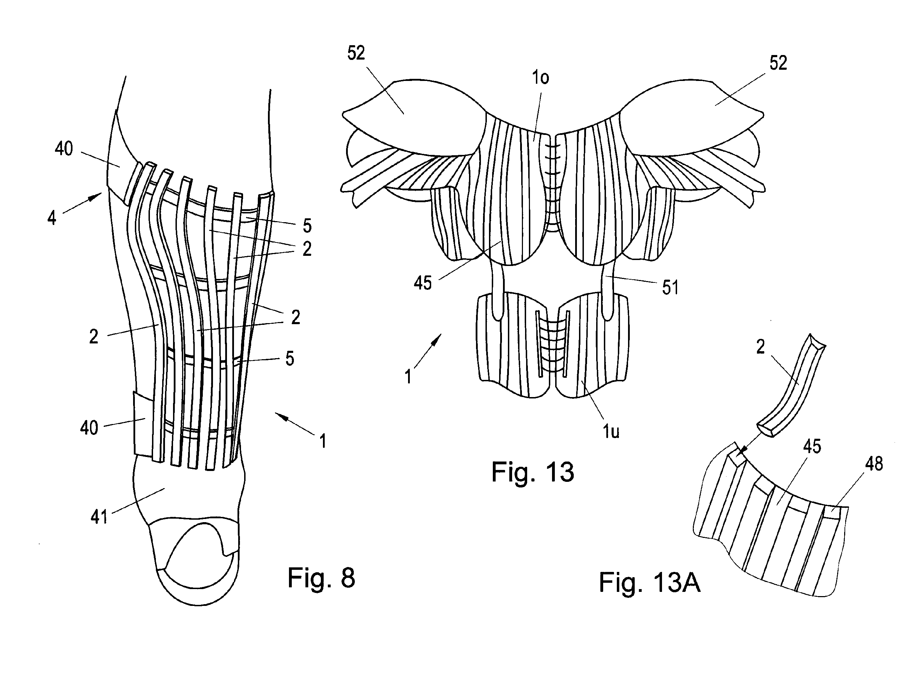

Anhand der Fig. 8 bis 15 sollen nun weitere Ausführungsbeispiele von Stütz- und Schutzeinrichtungen erläutert werden, die mit dem vorliegenden Verfahren in vorteilhafter Weise hergestellt werden können, wobei die vorstehend beschriebenen Grundprinzipien ebenso wie die vorgenannten Materialien bei der Herstellung zur Anwendung kommen können.

So zeigt Fig. 8 eine Stütz- bzw. Schutzeinrichtung 1 in Form eines Schienbein-Protektors, beispielsweise für Fußballspieler, die nach demselben Prinzip wie die Stützeinrichtung 1 gemäß Fig. 1 ausgebildet ist bzw. hergestellt wird. Die gleichartige Konstruktion ergibt sich bereits bei einem einfachen Vergleich der Fig. 1 und 8. Ein Unterschied liegt zwischen der Stützeinrichtung 1 gemäß Fig. 1 und der Schutzeinrichtung 1 gemäß Fig. 8 nur in der Anwendung insofern, als mit der Stützeinrichtung 1 gemäß Fig. 1 der jeweilige Körperteil nach einer Verletzung insgesamt gestützt (und auch geschützt) wird, um so den Körperteil zu entlasten bzw. zu schonen, wogegen im Fall der Schutzeinrichtung 1 gemäß Fig. 8 ein Schutz des jeweiligen Körperteils - hier des Unterschenkels - gegen etwaige Stöße und Schläge von außen, mit einer Druckverteilung über die Stütz- bzw. Schutzteile 2, erreicht werden soll.

Ähnlich wie gemäß Fig. 1 liegen auch gemäß Fig. 8 Stütz- oder Schutzteile 2 vor, die nach dem beschriebenen Verfahren individuell an den Träger bzw. dessen Schienbeinbereich angepasst werden, indem vom betroffenen Körperteil Bilddaten aufgenommen und zu einem 3D-Modell 13 (s. Fig. 2 und 3) verarbeitet werden, wobei die erhaltenen Daten dann zur Herleitung von Steuerdaten für die Form-Anpassung der Passteile 2 herangezogen werden. Die Fixiervorrichtung 4 weist in der Schutzeinrichtung 1 gemäß Fig. 8 außer den Bändern 5, die in entsprechender Weise wie vorstehend beschrieben vorliegen, und die insbesondere auch mit Distanzelementen, wie in den Fig. 4 bis 7 gezeigt, versehen sein können, die sich aber gemäß Fig. 8 nur über einen Teil des Umfangs des Unterschenkels erstrecken, beispielsweise am oberen Ende und am unteren Ende der Schutzeinrichtung 1 Fixier- oder Haltegurte 40 auf, die z.B. an dortige Bänder 5 anschließen, und die beispielsweise aus textilem Material mit relativ großer Breite bestehen und das Fixieren oder Schließen der Fixiervorrichtung 4 um den Unterschenkel herum gestatten. Dazu sind diese Fixier- oder Haltegurte 40 mit in Fig. 8 nicht näher ersichtlichen herkömmlichen Verschlüssen, wie etwa einem Klettverschluss, aber auch ggf. einem Verschluss mit Haken oder einer Schnalle, versehen.

Am unteren Ende der Schutzeinrichtung 1 kann gemäß Fig. 8 noch als Übergangsstück zum Fußbereich ein textiler Manschettenteil 41, z.B. aus einem dehnbaren Gewebe (Stretch-Gewebe) vorgesehen sein.

An sich könnte die Einrichtung 1 gemäß Fig. 8 auch als Stützeinrichtung ähnlich wie jene von Fig. 1 eingesetzt werden, etwa im Fall einer Schienbeinprellung, wobei es auch denkbar wäre, die Stützteile 2 nach unten über den Rist des Fußes in den Vorderfußteil zu verlängern.

Ein Unterschied zwischen den Einrichtungen von Fig. 1 und Fig. 8 besteht noch darin, dass bei der Einrichtung 1 von Fig. 1 die Bänder 5 außerhalb der Stützteile 2 veranschaulicht sind, wogegen gemäß Fig. 8 die Bänder 5 innerhalb der Stützteile 2, also näher dem Unterschenkel, angeordnet sind. Dies ist bei einer primären Schutzfunktion, nämlich als Schienbeinschützer, leichter möglich und vorteilhafter, wogegen bei einer wesentlichen Stützfunktion, wie gemäß Fig. 1, die Stützteile 2 möglichst eng am zu stützenden Körperteil angebracht sein sollen.

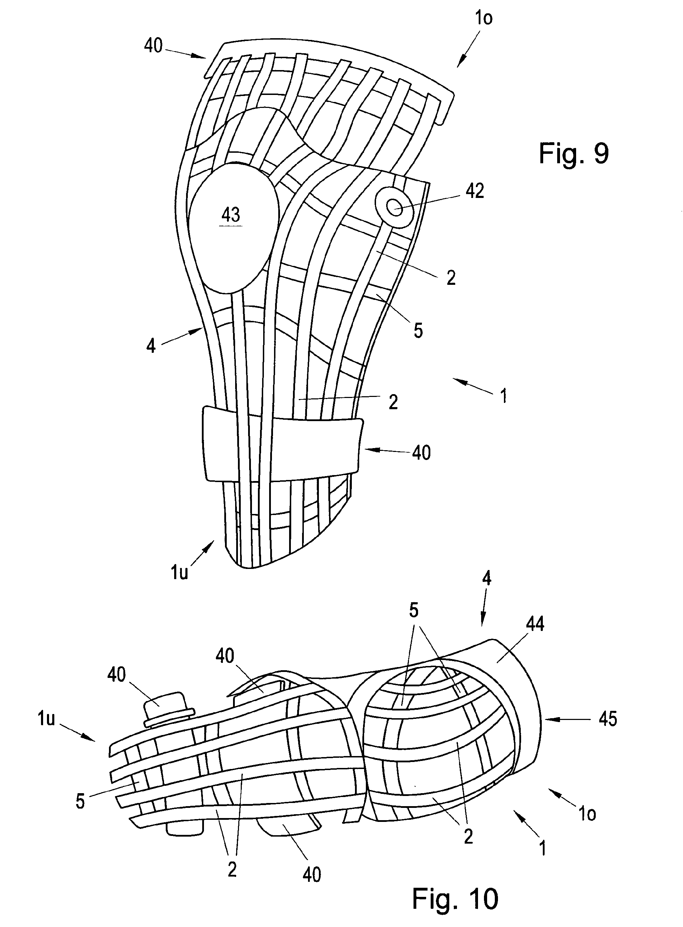

In Fig. 9 ist eine Anwendung der erfindungsgemäßen Technik ähnlich jener von Fig. 8 ersichtlich, nämlich für einen Bein-Protektor als Schutzeinrichtung 1, beispielsweise für Motorradfahrer. Auch hier liegt eine individuelle Formung der Schutzeinrichtung 1 zugrunde, wie vorstehend erläutert wurde, wobei insbesondere die Stütz- oder Schutzteile 2, allgemein Passteile 2, aufgrund des rechnerisch ermittelten 3D-Modells individuell angepasst werden. Die Passteile 2 sind wiederum, ähnlich wie gemäß Fig. 8, oder gemäß Fig. 1, durch quer verlaufende Bänder 5 miteinander verbunden und in ihrer Position fixiert, um so die Fixiervorrichtung 4 zu bilden. Zu dieser Fixiervorrichtung 4 gehören weiters noch ein oberes und ein unteres Umschließungsband als Fixier-Gurte 40. Der obere Fixier-Gurt 40 ist dabei an einem gesonderten Oberschenkel-Schutzbereich 1o angebracht, der mit dem Unterschenkel-Schutzbereich 1u über ein Gelenk 42 in an sich herkömmlicher Weise verbunden wird. Weiters ist am Unterschenkel-Schutzbereich 1u im vorderen Kniebereich eine Knieplatte 43 angebracht, die ebenfalls individuell geformt wird und so allgemein einen schalenförmigen Stütz- bzw. Schutzteil oder Passteil 2 bilden kann.

Fig. 10 veranschaulicht in einer weiteren schaubildlichen Darstellung einen individuell geformten Ellbogen-Protektor als Stütz- oder Schutzeinrichtung 1, wobei ein Unterarmteil 1u und ein zum Oberarm hinreichender Gelenkschutzteil 1o vorgesehen sind; diese beiden Schutzeinrichtungen 1u, 1o sind nach der vorstehend beschriebenen Technik hergestellt, wobei wiederum gemäß einem durch Fotografieren etc. sowie rechnerisches Ermitteln erhaltenen 3D-Modell individuell thermoplastisch umgeformte Stützoder Schutzteile bzw. Passteile 2 vorliegen, die durch zu ihnen quer verlaufende Bänder 5 verbunden und fixiert werden. Weiters sind wiederum Fixier-Gurte 40 vorgesehen. Darüber hinaus ist im Bereich des Ellbogengelenks eine Gewebelage 44 als eine Art Fortsetzung der Stützeinrichtung 1u, 1o, genauer von deren Passteilen 2, vorgesehen; diese Gewebelage 44 kann ähnlich wie der Manschettenteil 41 gemäß Fig. 8 als Abschluss für die Einrichtung 1 vorgesehen sein, es kann jedoch auch die Gewebelage 44 als Flächengebilde bzw. Unterlage 45 zwischen der eigentlichen Stütz- bzw. Schutzeinrichtung 1 und dem Körperteil vorliegen. Dieses Flächengebilde 45 ist an den Körperteil angepasst und ist so Teil der Fixiervorrichtung 4, in Verbindung mit den Bändern 5 sowie den Fixier-Gurten 40.

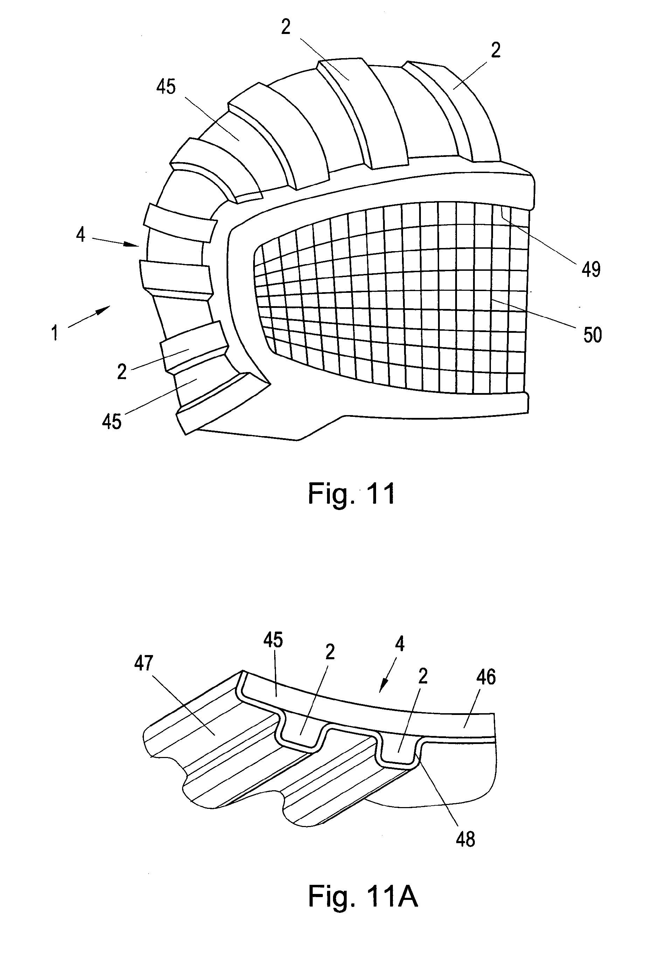

In Fig. 11 und 11A ist eine weitere Anwendung der erfindungsgemäßen Technik veranschaulicht, nämlich eine erfindungsgemäß hergestellte Schutzeinrichtung 1 in Form eines Schutzhelms für beispielsweise Eishockey- oder Football-Spieler oder für Motorradfahrer. Dieser Helm wird ebenfalls gemäß dem jeweiligen Träger individuell passend geformten Passteilen 2 hergestellt, die nunmehr an einer Schaumstoff-Unterlage 46, die dem Kopf des Trägers zugewandt ist, fixiert sind. Diese Schaumstoff-Unterlage 46 bildet wiederum ein Flächengebilde 45, und sie besteht, wie insbesondere aus Fig. 11A ersichtlich ist, aus einer vergleichsweise dicken Schaumstoff- oder Kautschuklage, wie etwa aus dem unter der Bezeichnung Neopren bekannten Chloropren-Material. Auf diesem Flächengebilde 45 sind, beispielsweise mit Hilfe eines flächig daran fixierten textilen Gewebes 47, Taschen 48 gebildet, in die die Passteile 2 eingeschoben werden. Dadurch bildet das Flächengebilde 45 mit der Chloroprenunterlage 46 und dem Taschen-Gewebe 47, mit den Taschen 48, in diesem Fall die Fixiervorrichtung 4 für die Schutzeinrichtung 1. Der gezeigte Helm gemäß Fig. 11 hat im Gesichtsbereich weiters eine Sichtöffnung 49, die beispielsweise in an sich herkömmlicher Weise mit einem Schutzgitter 50 verschlossen ist, durch das eine ausreichende Sicht gewährleistet ist, wobei andererseits Schutz gegen äußere Krafteinflüsse durch das Gitter 50 gegeben ist, wie dies an sich bekannt ist.

Gegebenenfalls kann über den Passteilen 2 noch ein Außenhelm, beispielsweise eine Hartschale, in an sich bekannter Weise angebracht werden.

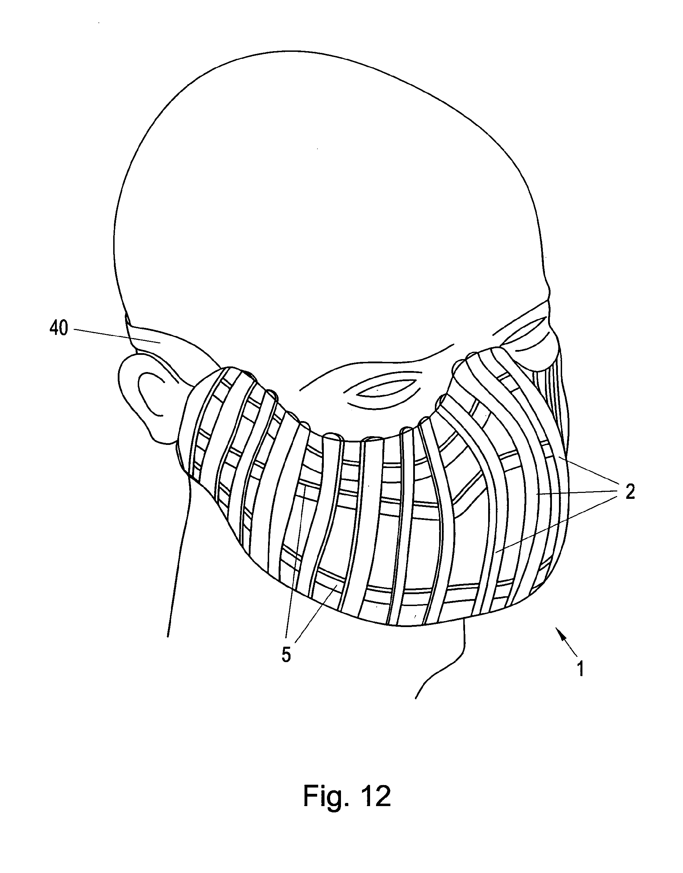

In Fig. 12 ist eine erfindungsgemäß hergestellte Schutzeinrichtung 1 in Form einer Schutzmaske, etwa für einen Eishockey-Tormann, veranschaulicht, wobei diese Schutzmaske auch gegebenenfalls mit einem Helm wie in Fig. 11 gezeigt kombiniert werden kann. Auch hier liegen wiederum individuell angepasste, anatomisch geformte Passteile 2 vor, die mit Hilfe von Bändern 5 (die u.U. auch mit Distanzelementen 9 bestückt sind) positioniert und fixiert werden. Die gesamte Maske, d.h. Schutzeinrichtung 1, wird weiters mit Gewebebändern, die als Fixier-Gurte 40 verwendet werden, am Kopf festgemacht.

In Fig. 13 und 13A ist eine Schutzeinrichtung 1 in Form einer Schutzkleidung, beispielsweise für (American) Football-Spieler, veranschaulicht, wobei die Schutzeinrichtung 1 gemäß Fig. 13 einen Oberteil 1o sowie einen gegebenenfalls damit über Bänder 51 verbundenen Unterteil 1u aufweist. Die Fixiervorrichtung 4 ist hier mit einem als Unterlage vorgesehenen Flächengebilde 45, nämlich einem textilen Gewebe, gebildet; dieses das Flächengebilde 45 bildende textile Gewebe, z.B. aus Kunstfaser, hat ähnlich wie die Unterlage 45-47 gemäß Fig. 11A Taschen 48, hier durch Nähen gebildete Taschen, siehe Fig. 13A, in die die wiederum an die individuelle Form des Körperteils des Trägers angepassten Passteile 2 - selbstverständlich in der richtigen Position, beispielsweise entsprechend einer Markierung gemäß der Einheit 21, Punkt 6 (Fig. 2 und 3) - eingesteckt werden. Zusätzlich können auch passend geformte Schulterteile 52 mit dem Flächengebilde 45 verbunden werden.

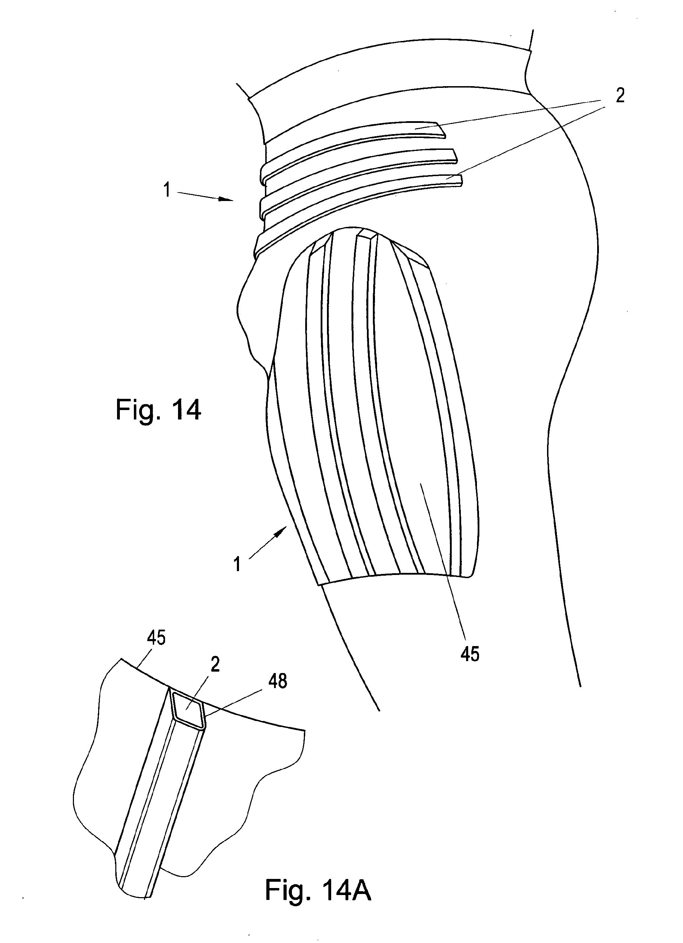

In Fig. 14 und 14A ist in ähnlicher, schaubildlicher Weise eine Schutzeinrichtung 1 in Form einer Schutzhose gezeigt, wobei wiederum in den zu schützenden Körperbereichen anatomisch individuell geformte Passteile 2 vorliegen, die in Taschen 48 in einem Gewebe-Flächengebilde 45 der Hose eingesteckt sind.

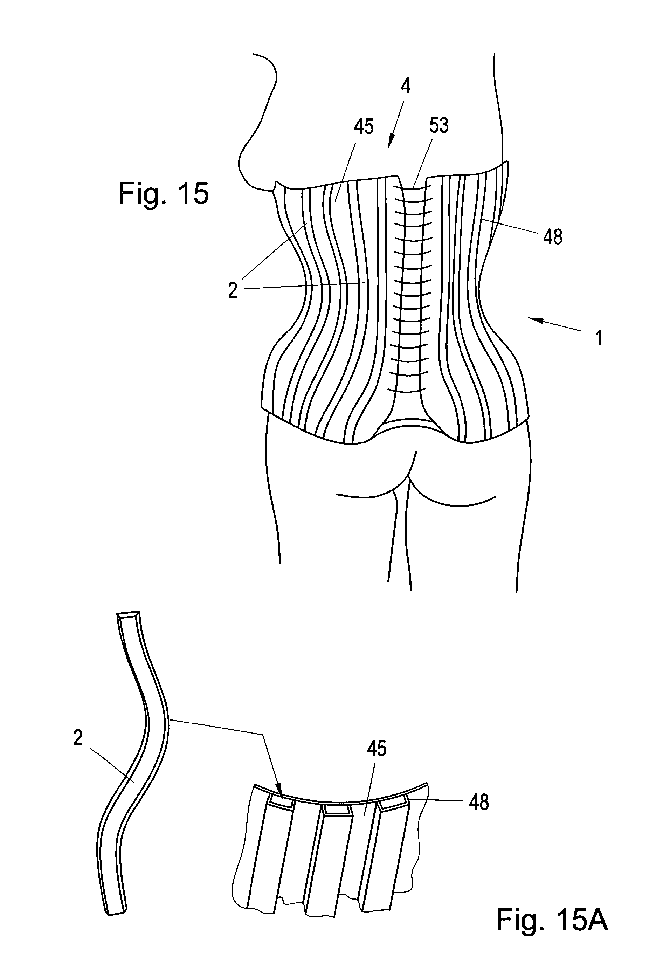

In entsprechender Weise kann auch die in Fig. 15 und 15A gezeigte Stützeinrichtung 1 erhalten werden, die hier ein Korsett, Mieder oder dergleichen bildet. Wiederum sind individuell angepasste Stütz- bzw. Passteile 2 vorhanden, die, wie vorstehend anhand der Fig. 2 und 3 erläutert, entsprechend dem rechnerisch erhaltenen 3D-Modell umgeformt wurden. Diese Passteile 2 werden wiederum in genähte Taschen 48 an einem Gewebe-Flächengebilde 45 eingesteckt. Das Flächengebilde 45 bildet hier zusammen mit einem Verschluss, der mit herkömmlichen Haken 53 ausgeführt ist, die Fixiervorrichtung 4 dieser Stützeinrichtung 1.

Die Passteile 2 werden im Fall der Stützeinrichtung 1 gemäß Fig. 15 und 15A als eher flache, leistenförmige Stützteile ausgebildet, im Gegensatz etwa zu den Stützteilen 2 gemäß Fig. 1 oder den Passteilen 2 gemäß Fig. 8 oder 9.

Selbstverständlich sind noch weitere Anwendungsmöglichkeiten für die erfindungsgemäße Technik gegeben; beispielsweise ist es auch denkbar, eine Stützeinrichtung wie einen Sattel, z.B. Fahrradsattel, oder auch Reitsattel (bei dessen Formgebung nicht nur Reiter, sondern auch vom Pferderücken Bilddaten aufgenommen werden können, so dass der Vorteil einer anatomisch günstigen gleichmäßigen Gewichtsverteilung des Reiters auf dem Pferderücken ermöglicht wird) oder anatomisch geformte Fahrzeugsitze auf die beschriebene Weise mit individueller Anpassung an die Körperform des Benützers herzustellen.

## Patentansprüche

1. Verfahren zum Herstellen einer Stütz- oder Schutzeinrichtung (1) für einen Körperteil, welche zumindest einen in der Form an den Körperteil angepassten Stütz- oder Schutzteil (2) aufweist, der mittels einer Fixiervorrichtung (4) an den Körperteil individuell anpassbar und/oder an ihm befestigbar ist, wobei vom Körperteil Bilddaten aufgenommen und zur Ermittlung eines 3D-Modells (13) mit Hilfe von Rechnermitteln (19) ausgewertet werden, und aus dem 3D-Modell Steuerdaten für die Form-Anpassung des Stütz- oder Schutzteils (2) hergeleitet werden, **dadurch gekennzeichnet, dass** als Stütz- oder Schutzteile (2) stab- bzw. leistenförmige Stützelemente (3) vorgesehen werden, die aus einem plastisch verformbaren bzw. einem Material mit Memory-Effekt bestehen, und die auf Basis der hergeleiteten Steuerdaten maschinell verformt werden.

2. Verfahren nach Anspruch 1, **dadurch gekennzeichnet, dass** das Aufnehmen von Bilddaten die Herstellung von Röntgen- oder CT-Aufnahmen, z.B. bei Knochenbrüchen, Missbildungen oder Fehlstellungen, umfasst.

3. Verfahren nach Anspruch 1 oder 2, **dadurch gekennzeichnet, dass** aus dem 3D-Modell auch Steuerdaten für eine Anpassung der Fixiervorrichtung (4) an den Körperteil hergeleitet werden.

4. Verfahren nach einem der Ansprüche 1 bis 3, **dadurch gekennzeichnet, dass** zur Bildung der Fixiervorrichtung (4) zumindest ein in der Form bzw. Länge angepasstes Band (5), vorzugsweise mit voneinander beabstandeten Distanzelementen (9), zwischen denen der zumindest eine Stützteil (2) eingefügt wird, angefertigt wird, wobei die Distanzelemente vorzugsweise in einem von den Steuerdaten vorgegebenen Abstand am Band (5) befestigt, z.B. aufgeklebt und/oder aufgeklipst, oder gegebenenfalls in einem von den Steuerdaten vorgegebenen Abstand am Band (5) durch Ausfräsen hergestellt werden.

5. Verfahren nach Anspruch 4, **dadurch gekennzeichnet, dass** der oder die Stütz- oder Schutzteile (2) nach der Formung am Band (5) befestigt, z.B. aufgeklipst, angeklebt oder US-geschweißt, werden.

6. Verfahren nach einem der Ansprüche 1 bis 5, **dadurch gekennzeichnet, dass** zur Bildung der Fixiervorrichtung (4) ein an den Körperteil angepasstes Flächengebilde (45), z.B. ein textiles Gewebe, angefertigt wird, welches Flächengebilde (45) vorzugsweise als Unterlage für den oder die Stütz- oder Schutzteile (2), gegebenenfalls samt Fixier-Band (5), angefertigt wird.

7. Verfahren nach Anspruch 6, **dadurch gekennzeichnet, dass** das Flächengebilde (45) mit einer oder mehreren Taschen (48) versehen wird, in die der oder die Stütz- oder Schutzteile (2) eingesteckt wird bzw. werden.

8. Verfahren nach einem der Ansprüche 1 bis 7, **dadurch gekennzeichnet, dass** die Fixiervorrichtung (4) mit gesonderten Fixier-Gurten (40), Haken (53) oder dergleichen Verschlussteilen zum Fixieren der Stütz- oder Schutzeinrichtung (1) am Körperteil ausgerüstet wird.

9. Verfahren nach einem der Ansprüche 1 bis 8, **dadurch gekennzeichnet, dass** die Stütz- oder Schutzelemente (3) von einem Stangenmaterial auf Basis der Steuerdaten maschinell in der erforderlichen Länge abgeschnitten werden, bevor oder nachdem sie verformt werden.

10. Verfahren nach einem der Ansprüche 1 bis 9, **dadurch gekennzeichnet**, das der oder die aus Kunststoff bestehenden Stütz- oder Schutzteile (2), gegebenenfalls auch die Distanzelemente (9), durch Anwendung von Hitze und Druck, Kälte, Säge-, Laser- und/oder Fräseinrichtungen (21.5) auf Basis der Steuerdaten maschinell verformt werden.

11. Verfahren nach einem der Ansprüche 1 bis 10, **dadurch gekennzeichnet, dass** die Stütz- oder Schutzteile (2) zwecks Festlegung ihrer Position an der Fixiervorrichtung (4) auf Basis der Steuerdaten automatisch markiert werden.

12. Verfahren nach einem der Ansprüche 1 bis 11, **dadurch gekennzeichnet, dass** die Oberfläche des Stütz- oder Schutzteils (2) und/oder des Bandes (5) und der Distanzelemente (9) mit einer Lotuseffekt-Nanostruktur ausgebildet wird.

13. Verfahren nach einem der Ansprüche 1 bis 12, **dadurch gekennzeichnet, dass** zur Bilddaten-Aufnahme eine digitale Kamera (10), eine Phasenkamera oder ein Laserscanner verwendet wird.

14. Verfahren nach einem der Ansprüche 1 bis 13, **dadurch gekennzeichnet, dass** das 3D-Modell unter Anwendung eines an sich bekannten Konturlinienverfahrens erstellt wird.

15. Verfahren nach einem der Ansprüche 1 bis 14, **dadurch gekennzeichnet, dass** bei der Ermittlung des 3D-Modells auch über eine Eingabeeinrichtung (25) eingegebenen individuellen Korrektur-Eingaben berücksichtigt werden.

## Claims

1. A method for producing a supporting or protective device (1) for a body part, comprising at least one supporting or protective member (2) adapted in shape to the body part and designed to be individually adaptable and/or attachable to the body part by means of a fixing device (4), wherein image data are taken from the body part and are evaluated with the aid of computing means (19) for determining a 3D model (13), and wherein control data for the adaptation of the shape of the supporting or protective member (2) are derived from the 3D model, **characterized in that** rod or slat shaped support elements (3) consisting of a plastically deformable material and/or a material with memory effect are provided as supporting or protective members (2), which are deformed mechanically on the basis of the control data derived.

2. The method according to claim 1, **characterized in that** the taking of image data comprises the production of x-ray or computer tomography pictures, e.g. in the case of bone fractures, abnormalities or defective positions.

3. The method according to claims 1 or 2, **characterized in that** control data for adapting the fixing device (4) to the body part are also derived from the 3D model.

4. The method according to any of claims 1 to 3, **characterized in that**, for forming the fixing device (4), at least one band (5) adapted in shape and/or length is manufactured, preferably with distance elements (9) which are spaced apart from each other and between which the at least one supporting member (2) is inserted, wherein the distance elements are preferably fixed, e.g. glued and/or clipped, on the band (5) at a distance predetermined by the control data, or are possibly produced on the band (5) by means of milling at a distance predetermined by the control data.

5. The method according to claim 4, **characterized in that** the supporting or protective member(s) (2) is/are fixed, e.g. clipped, glued or US-welded, on the band (5) after shaping.

6. The method according to any of claims 1 to 5, **characterized in that**, for forming the fixing device (4), a flat shaped article (45), e.g. a textile tissue, adapted to the body part is manufactured, said fabric (45) preferably being manufactured as a pad for the supporting or protective member(s) (2), possibly along with the fixing band (5).

7. The method according to claim 6, **characterized in that** the fabric (45) is provided with one or a plurality of pockets (48) into which the supporting or protective member(s) (2) is/are inserted.

8. The method according to any of claims 1 to 7, **characterized in that** the fixing device (4) is equipped with separate fixing belts (40), hooks (53) or similar locking members for fixing the supporting or protective device (1) on the body part.

9. The method according to any of claims 1 to 8, **characterized in that** the supporting or protective elements (3) are mechanically cut from a rod material with the required length on the basis of the control data before or after being deformed.

10. The method according to any of claims 1 to 9, **characterized in that** the supporting or protective member(s) (2) consisting of plastics, possibly also the distance elements (9), are mechanically deformed on the basis of the control data by the use of heat and pressure, coldness, sawing, laser and/or milling devices (21.5).

11. The method according to any of claims 1 to 10, **characterized in that** the supporting or protective members (2) are marked automatically on the basis of the control data for the purpose of determining their position at the fixing device (4).

12. The method according to any of claims 1 to 11, **characterized in that** the surface of the supporting or protective member (2) and/or of the band (5) and the distance elements (9) is formed with a lotus effect nano structure.

13. The method according to any of claims 1 to 12, **characterized in that** a digital camera (10), a phase camera or a laser scanner is used for taking the image data.

14. The method according to any of claims 1 to 13, **characterized in that** the 3D model is produced by using a per se known contour line method.

15. The method according to any of claims 1 to 14, **characterized in that** individual correction data input via an input device (25) are also taken into account during the determination of the 3D model.

## Revendications

1. Procédé permettant la fabrication d'un dispositif de soutien ou de protection (1) pour une partie du corps, qui comporte au moins une partie de soutien ou de protection (2), qui est ajustée à la forme de la partie du corps et qui peut être adaptée individuellement à la partie du corps au moyen d'un dispositif de fixation (4) et/ou peut être fixée à ladite partie du corps, des données d'image de la partie du corps étant enregistrées et étant analysées pour établir un modèle 3D (13) à l'aide de moyens de calcul (19), et des données de contrôle pour l'ajustement de la forme de la partie de soutien ou de protection (2) étant déduites du modèle 3D, **caractérisé en ce que** pour former les parties de soutien ou de protection (2), il est prévu des éléments de soutien (3) en forme de tiges ou baguettes, qui sont réalisés dans un matériau à déformation plastique ou un matériau à effet de mémoire et qui sont formés mécaniquement sur la base des données de contrôle déduites.

2. Procédé selon la revendication 1, **caractérisé en ce que** l'enregistrement de données d'image comporte la réalisation d'enregistrements par rayons X ou par tomodensitométrie, par exemple en cas de fractures osseuses, de malformations ou de difformités.

3. Procédé selon la revendication 1 ou 2, **caractérisé en ce que** des données de contrôle pour un ajustement du dispositif de fixation (4) à la partie du corps peuvent aussi être déduites du modèle 3D.

4. Procédé selon l'une quelconque des revendications 1 à 3, **caractérisé en ce que** pour former le dispositif de fixation (4), on réalise au moins une bande (5) ajustée à la forme ou à la longueur, de préférence des éléments d'écartement (9) écartés les uns des autres, entre lesquels est insérée ladite au moins une partie de soutien (2), les éléments d'écartement étant fixés, par exemple collés et/ou clipsés, sur la bande (5) à une distance définie par les données de contrôle, ou, le cas échéant, étant réalisés par fraisage sur la bande (5) à une distance prédéfinie par les données de contrôle.

5. Procédé selon la revendication 4, **caractérisé en ce que** la ou les parties de soutien ou de protection (2) sont fixées, par exemple clipsées, collées ou soudées par ultrasons, sur la bande (5) après le formage.

6. Procédé selon l'une quelconque des revendications 1 à 5, **caractérisé en ce que** pour former le dispositif de fixation (4), on réalise une formation de surface (45), par exemple une structure textile, ajustée à la partie du corps, ladite formation de surface (45) étant réalisée de préférence sous forme de sous-couche pour la ou les parties de soutien ou de protection (2), le cas échéant conjointement avec la bande de fixation (5).

7. Procédé selon la revendication 6, **caractérisé en ce que** la formation de surface (45) est munie d'une ou de plusieurs poches (48) dans laquelle est enfichée ou sont enfichées la ou les parties de soutien ou de protection (2).

8. Procédé selon l'une quelconque des revendications 1 à 7, **caractérisé en ce que** le dispositif de fixation (4) est muni de sangles de fixation (40) séparées, crochets (53) ou éléments de fermeture similaires pour fixer le dispositif de soutien ou de protection (1) sur la partie du corps.

9. Procédé selon l'une quelconque des revendications 1 à 8, **caractérisé en ce que** les éléments de soutien ou protection (3), avant ou après qu'ils aient été formés, sont coupés mécaniquement à la longueur requise dans un matériau pour tiges, sur la base des données de contrôle.

10. Procédé selon l'une quelconque des revendications 1 à 9, **caractérisé en ce que** la ou les parties de soutien ou de protection (2) réalisées en matière plastique, le cas échéant aussi les éléments d'écartement (9) sont formés mécaniquement sur la base des données de contrôle, sous l'effet d'une application de chaleur et pression, de froid, sur des dispositifs de sciage, des dispositifs laser et/ou des dispositifs de fraisage (21.5).

11. Procédé selon l'une quelconque des revendications 1 à 10, **caractérisé en ce que**, en vue de la détermination de leur position sur le dispositif de fixation (4), les parties de soutien ou de protection (2) sont marqués automatiquement sur la base des données de contrôle.

12. Procédé selon l'une quelconque des revendications 1 à 11, **caractérisé en ce que** la surface de la partie de soutien ou de protection (2) et/ou de la bande (5) et des éléments d'écartement (9) est réalisée avec une nanostructure à effet lotus.

13. Procédé selon l'une quelconque des revendications 1 à 12, **caractérisé en ce que** pour l'enregistrement des données d'image, on utilise une caméra numérique (10), une caméra à rafale ou un scanner laser.

14. Procédé selon l'une quelconque des revendications 1 à 13, **caractérisé en ce que** le modèle 3D est établi moyennant l'utilisation d'un procédé de lignes de contour connu en soi.

15. Procédé selon l'une quelconque des revendications 1 à 14, **caractérisé en ce qu'**au moment de l'établissement du modèle 3D, on tient compte d'entrées correctives individuelles, également entrées par l'intermédiaire d'un dispositif d'entrée (25).
